# EUROPÄISCHE PATENTSCHRIFT

(11) **EP 3 834 754 B1**
(45) Veröffentlichungstag und Bekanntmachung des Hinweises auf die Patenterteilung: **31.05.2023**
(21) Anmeldenummer: 20212741.1
(22) Anmeldetag: 09.12.2020
(51) Int. Cl.: A61B 17/3201, A61B 17/32

(54) **MEDIZINISCHE SCHERE FÜR MIKROINVASIVE ANWENDUNGEN**
MEDICAL SCISSORS FOR MICRO-INVASIVE APPLICATIONS
CISEAUX MÉDICAUX POUR APPLICATIONS MICRO-INVASIVES

(30) Priorität: 11.12.2019 DE 102019134017
(43) Veröffentlichungstag der Anmeldung: 16.06.2021
(73) Patentinhaber: Karl Storz SE & Co. KG, 78532 Tuttlingen (DE)
(72) Erfinder: Stefan, Jochen, 78532 Tuttlingen (DE)

(56) Entgegenhaltungen:
- EP-A1- 3 213 702
- WO-A1-2019/050025
- JP-A- 2013 138 844

## Beschreibung

Die vorliegende Erfindung ist auf eine medizinische Schere für mikroinvasive Anwendungen und ein medizinisches Instrument mit einer solchen Schere bezogen.

Medizinische Scheren für mikroinvasive Anwendungen müssen besondere und besonders hohe Anforderungen erfüllen. Diese ergeben sich einerseits aus den geringen Abmessungen, die an sich schon eine Übertragung von Konzepten, die bei deutlich größeren Scheren bewährt sind, oft erschweren oder ausschließen. Hinzu kommen andererseits Forderungen nach einer besonderen Zuverlässigkeit, die aus der besonderen Anwendungssituation resultieren. Angesichts der deutlich weniger unmittelbaren mechanischen Kontrolle und der deutlich weniger unmittelbaren mechanischen Rückmeldung soll eine medizinische Schere für mikroinvasive Anwendungen zuverlässig bei jedem Schließen schneiden. Ein Einklemmen des Schnittguts zwischen den Scherenblättern statt eines Schneidens oder ein Wegrutschen des Schnittguts nach distal kann nicht nur die Dauer einer mikroinvasiven Maßnahme verlängern, sondern im Einzelfall durchaus die Gesundheit des Patienten gefährden.

In EP 3 213 702 A1 ist eine endoskopische Schere beschrieben. Schneidkanten von Scherenblättern weisen konkave Abschnitte auf.

In WO 2019/050025 A1, auch veröffentlicht als EP 3 682 833 A1, ist eine medizinische Schere beschrieben. Schneidkanten der Scherenblätter weisen Ausnehmungen zwischen vorspringenden Abschnitten auf.

Eine Aufgabe der vorliegenden Erfindung besteht darin, eine verbesserte medizinische Schere für mikroinvasive Anwendungen und ein verbessertes medizinisches Instrument mit einer solchen Schere zu schaffen.

Diese Aufgabe wird durch den Gegenstand des unabhängigen Anspruchs gelöst.

Weiterbildungen sind in den abhängigen Ansprüchen angegeben.

Ausführungsformen der vorliegenden Erfindung beruhen auf der Idee, die Eigenschaften der Schneidkanten der Scherenblätter, insbesondere die Keilwinkel oder die Schneidwinkel oder die Breiten der Biseau-Flächen oszillierend, also mehrfach abwechselnd zu- und abnehmend auszugestalten.

Eine medizinische Schere für mikroinvasive Anwendungen umfasst ein erstes Scherenblatt mit einer ersten Schneidkante zwischen einer Freifläche und einer Biseau-Fläche, ein relativ zu dem ersten Scherenblatt bewegbares zweites Scherenblatt mit einer zweiten Schneidkante zwischen einer Freifläche und einer Biseau-Fläche und eine Führungseinrichtung zur mechanischen Führung des zweiten Scherenblatts relativ zu dem ersten Scherenblatt derart, dass die zweite Schneidkante die erste Schneidkante jederzeit in einem Schnittpunkt berührt, wobei die Führungseinrichtung mit einem Schaft eines mikroinvasiven Instruments starr verbunden oder verbindbar ist, wobei die erste Schneidkante durch einen ersten Parameter charakterisiert ist, der entlang der ersten Schneidkante mehrfach zunimmt und mehrfach abnimmt, wobei die zweite Schneidkante durch einen zweiten Parameter charakterisiert ist, der entlang der zweiten Schneidkante mehrfach zunimmt und mehrfach abnimmt, wobei der erste Parameter und der zweite Parameter jeweils der Keilwinkel zwischen Freifläche und Biseau-Fläche oder der Schneidwinkel oder die Breite der Biseau-Fläche ist.

Die Schneidkanten sind konvexe, scharfe Kanten, die bei Scheren auch als Waten bezeichnet werden. Der Schneidwinkel ist die Summe aus Freiwinkel und Keilwinkel. Die Biseau-Fläche ist die an die Schneidkante unmittelbar angrenzende ebene oder schwach gekrümmte Fläche.

Die Führungseinrichtung und die Schneidkanten sind so ausgebildet und angeordnet, dass die Schneidkanten einander jederzeit in genau einem Schnittpunkt berühren.

Die Führungseinrichtung ist beispielsweise ein Gelenk, das formschlüssig ausschließlich ein Schwenken des zweiten Scherenblatts relativ zu dem ersten Scherenblatt um eine durch das Gelenk formschlüssig definierte Schwenkachse ermöglicht. Das Gelenk kann ein Schwenken beider Scherenblätter um eine gemeinsame oder um zwei verschiedene Schwenkachsen relativ zu dem distalen Ende des Schafts ermöglichen. Alternativ kann das erste Scherenblatt starr, also unbewegbar mit dem distalen Ende eines Schafts verbindbar oder verbunden sein.

Die Führungseinrichtung ist insbesondere zerstörungsfrei lösbar mit dem distalen Ende des Schafts mechanisch verbunden oder verbindbar. Alternativ kann die Führungseinrichtung mit dem distalen Ende des Schafts dauerhaft und nicht zerstörungsfrei lösbar verbunden sein.

Entlang der ersten Schneidkante nimmt der erste Parameter insbesondere abwechselnd mindestens zweimal zu und zweimal ab. Entlang der zweiten Schneidkante nimmt der zweite Parameter insbesondere abwechselnd mindestens zweimal ab und zweimal zu. Der erste Parameter und der zweite Parameter können sich entlang den Schneidkanten jeweils kontinuierlich oder diskontinuierlich, d. h. in Sprüngen, verändern. Der erste Parameter und der zweite Parameter können jeweils zwischen einem vorbestimmten Minimum und einem vorbestimmten Maximum und mit einer konstanten räumlichen Periode oszillieren. Alternativ können die Variationen des ersten Parameters und des zweiten Parameters sowohl in der Periode als auch in der Amplitude, also der Differenz benachbarter Minima und Maxima, variieren. Auch die Ortsabhängigkeit und die Veränderungsraten der Parameter können sich entlang den Schneidkanten wiederholen oder nicht wiederholen.

An der ersten Schneidkante und an der zweiten Schneidkante können der gleiche Parameter oder verschiedene Parameter variieren. Beispielsweise können an beiden Schneidkanten der Keilwinkel oder an beiden Schneidkanten der Schneidwinkel oder an beiden Schneidkanten die Breiten der Biseau-Flächen variieren. Alternativ können beispielsweise an einer Schneidkante der Keilwinkel und an der anderen Schneidkante die Breite der Biseau-Fläche variieren.

Bei einer medizinischen Schere, wie sie hier beschrieben ist, weist insbesondere die erste Schneidkante einen ersten Abschnitt, in dem der erste Parameter nicht größer ist als ein erster vorbestimmter Wert, und einen zweiten Abschnitt, in dem der erste Parameter nicht kleiner ist als ein zweiter vorbestimmter Wert, der größer ist als der erste vorbestimmte Wert, auf, wobei die zweite Schneidkante einen ersten Abschnitt, in dem der zweite Parameter nicht größer ist als ein dritter vorbestimmter Wert, und einen zweiten Abschnitt, in dem der zweite Parameter nicht kleiner ist als ein vierter vorbestimmter Wert, der größer ist als der dritte vorbestimmte Wert, aufweist, wobei der zweite Abschnitt der ersten Schneidkante dem ersten Abschnitt der zweiten Schneidkante gegenüberliegt und der zweite Abschnitt der zweiten Schneidkante dem ersten Abschnitte der ersten Schneidkante gegenüberliegt.

Bei einer medizinischen Schere, wie sie hier beschrieben ist, weist insbesondere die erste Schneidkante abwechselnd erste Abschnitte, in denen der erste Parameter nicht größer als ein erster vorbestimmter Wert, und zweite Abschnitte, in denen der erste Parameter nicht kleiner ist als ein zweiter vorbestimmter Wert, der größer ist als der erste vorbestimmte Wert, auf, wobei die zweite Schneidkante abwechselnd erste Abschnitte, in denen der zweite Parameter nicht größer ist als ein dritter vorbestimmter Wert, und zweite Abschnitte, in denen der zweite Parameter nicht kleiner ist als ein vierter vorbestimmter Wert, der größer ist als der dritte vorbestimmte Wert, ist, aufweist.

Bei einer medizinischen Schere, wie sie hier beschrieben ist, weist insbesondere die erste Schneidkante abwechselnd erste Abschnitte, in denen der erste Parameter nicht größer als ein erster vorbestimmter Wert, und zweite Abschnitte, in denen der erste Parameter nicht kleiner ist als ein zweiter vorbestimmter Wert, der größer ist als der erste vorbestimmte Wert, auf, wobei die zweite Schneidkante abwechselnd erste Abschnitte, in denen der zweite Parameter nicht größer ist als ein dritter vorbestimmter Wert, und zweite Abschnitte, in denen der zweite Parameter nicht kleiner ist als ein vierter vorbestimmter Wert, der größer ist als der dritte vorbestimmte Wert, ist, aufweist, wobei jeder zweite Abschnitt der ersten Schneidkante einem ersten Abschnitt der zweiten Schneidkante gegenüberliegt und jeder zweite Abschnitt der zweiten Schneidkante einem ersten Abschnitt der ersten Schneidkante gegenüberliegt.

Der erste vorbestimmte Wert ist insbesondere der kleinste Wert, den der erste Parameter entlang der gesamten ersten Schneidkante aufweist. Alternativ kann der erste Parameter innerhalb des ersten Abschnitts oder der ersten Abschnitte der ersten Schneidkante und alternativ oder zusätzlich außerhalb des ersten Abschnitts oder der ersten Abschnitte der ersten Schneidkante einen Werte anehmen, der kleiner ist als der erste vorbestimmte Wert. Der zweite vorbestimmte Wert ist insbesondere der größte Wert, den der erste Parameter entlang der gesamten ersten Schneidkante annimmt. Alternativ kann der zweite Parameter innerhalb des zweiten Abschnitts oder der zweiten Abschnitte der ersten Schneidkante und alternativ oder zusätzlich außerhalb des zweiten Abschnitts oder der zweiten Abschnitte der ersten Schneidkante einen Werte annehmen, der größer ist als der zweite vorbestimmte Wert.

Der vorbestimmte dritte Wert ist insbesondere der kleinste Wert, den der zweite Parameter entlang der gesamten zweiten Schneidkante annimmt. Alternativ kann der zweite Parameter innerhalb des ersten Abschnitts oder der ersten Abschnitte der zweiten Schneidkante und alternativ oder zusätzlich außerhalb des ersten Abschnitts oder der ersten Abschnitte der zweiten Schneidkante einen Wert annehmen, der kleiner ist als der dritte vorbestimmte Wert. Der vierte vorbestimmte Wert ist insbesondere der größte Wert, den der zweite Parameter entlang der gesamten zweiten Schneidkante annimmt. Alternativ kann der zweite Parameter innerhalb des zweiten Abschnitts oder der zweiten Abschnitte der zweiten Schneidkante und alternativ oder zusätzlich außerhalb des zweiten Abschnitts oder der zweiten Abschnitte der zweiten Schneidkante einen Wert annehmen, der größer ist als der vierte vorbestimmte Wert.

Bei einer medizinischen Schere, wie sie hier beschrieben ist, weist insbesondere die erste Schneidkante abwechselnd erste Abschnitte, in denen der erste Parameter kleiner ist als ein erster vorbestimmter Wert und zweite Abschnitte, in denen der erste Parameter größer ist als der erste vorbestimmte Wert, auf, wobei die zweite Schneidkante abwechselnd erste Abschnitte, in denen der zweite Parameter kleiner ist als ein dritter vorbestimmter Wert, und zweite Abschnitte, in denen der zweite Parameter größer ist als der dritte vorbestimmte Wert, aufweist, wobei jeder zweite Abschnitt der ersten Schneidkante einem ersten Abschnitt der zweiten Schneidkante gegenüberliegt und jeder zweite Abschnitt der zweiten Schneidkante einem ersten Abschnitt der ersten Schneidkante gegenüberliegt.

Unmittelbar benachbarte erste und zweite Abschnitte der ersten Schneidkante können unmittelbar aneinander anschließen oder durch - im mathematischen Sinne punktförmige - Bereiche, in denen der erste Parameter weder kleiner noch größer als, sondern gleich dem ersten vorbestimmten Wert ist, getrennt sein. Entsprechendes gilt für die ersten und zweiten Abschnitte der zweiten Schneidkante.

Die erste Schneidkante weist insbesondere zwei, drei, vier oder mehr erste Abschnitte und zwei, drei, vier oder mehr zweite Abschnitte auf. Die zweite Schneidkante weist insbesondere zwei, drei, vier oder mehr erste Abschnitte und zwei, drei, vier oder mehr zweite Abschnitte auf.

Ein Punkt an der ersten Schneidkante korrespondiert zu einem Punkt an der zweiten Schneidkante, wenn eine Konfiguration der medizinischen Schere existiert, bei der gleichzeitig der Punkt an der ersten Schneidkante und der Punkt an der zweiten Schneidkante mit dem Schnittpunkt der Schneidkanten identisch sind. Ein Abschnitt der ersten Schneidkante liegt einem Abschnitt der zweiten Schneidkante gegenüber, wenn zu Punkten innerhalb des Abschnitts der zweiten Schneidkante korrespondierende Punkte innerhalb des Abschnitts der ersten Schneidkante mindestens die Hälfte oder mindestens zwei Drittel oder mindestens drei Viertel des Abschnitts der ersten Schneidkante einnehmen und gleichzeitig zu Punkten innerhalb des Abschnitts der ersten Schneidkante korrespondierende Punkte innerhalb des Abschnitts der zweiten Schneidkante einen entsprechenden Anteil des Abschnitts der zweiten Schneidkante einnehmen.

Erste Abschnitte der ersten Schneidkante und erste Abschnitte der zweiten Schneidkante schneiden aufgrund des kleineren Keilwinkels und/oder des kleineren Schneidwinkels und/oder der geringeren Breite der Biseau-Fläche leichter oder besser als zweite Abschnitte der ersten Schneidkante und zweite Abschnitte der zweiten Schneidkante. Indem jedem zweiten Abschnitt der ersten Schneidkante ein erster Abschnitt der zweiten Schneidkante und jedem zweiten Abschnitt der zweiten Schneidkante ein erster Abschnitt der ersten Schneidkante gegenüberliegt, weist die medizinische Schere entlang den Schneidkanten insbesondere durchgehend oder mit geringen Abständen zumindest entweder gut schneidende erste Abschnitte der ersten Schneidkante oder gut schneidende erste Abschnitte der zweiten Schneidkante auf. Gleichzeitig kann die Profilierung der Schneidkanten durch die abwechselnde Anordnung erster und zweiter Abschnitte ein Herausrutschen eines zu schneidenden Gegenstands nach distal verhindern oder zumindest das Risiko dazu reduzieren.

Bei einer medizinischen Schere, wie sie hier beschrieben ist, sind die ersten Abschnitte und die zweiten Abschnitte der ersten Schneidkante und die ersten Abschnitte und die zweiten Abschnitte der zweiten Schneidkante insbesondere so angeordnet, dass beim Schließen der medizinischen Schere der Schnittpunkt jederzeit zumindest entweder an einem ersten Abschnitt der ersten Schneidkante oder an einem ersten Abschnitt der zweiten Schneidkante liegt.

Dies kann eine durchgehend gute Schneidwirkung der medizinischen Schere ermöglichen.

Bei einer medizinischen Schere, wie sie hier beschrieben ist, sind die ersten Abschnitte und die zweiten Abschnitte der ersten Schneidkante und die ersten Abschnitte und die zweiten Abschnitte der zweiten Schneidkante insbesondere so angeordnet sind, dass beim Schlie-ßen der medizinischen Schere Bereiche, in denen der Schnittpunkt weder an einem ersten Abschnitt der ersten Schneidkante noch an einem ersten Abschnitt der zweiten Schneidkante liegt, nicht größer sind als benachbarte Bereiche, in denen der Schnittpunkt an einem ersten Abschnitt der ersten Schneidkante oder an einem ersten Abschnitt der zweiten Schneidkante liegt.

Mit Bereichen sind insbesondere Bereiche des Winkels zwischen den Scherenblättern oder Bereiche der Koordinate des Schnittpunkts entlang einer der beiden Schneidkanten gemeint.

Durch die kurzen Unterbrechungen zwischen Bereichen, in denen der Schnittpunkt entweder an einem ersten Abschnitt der ersten Schneidkante oder an einem ersten Abschnitt der zweiten Schneidkante liegt, wird die besonders gute Schneideigenschaft nur kurz unterbrochen.

Bei einer medizinischen Schere, wie sie hier beschrieben ist, ist insbesondere zumindest entweder in jedem ersten Abschnitt der ersten Schneidkante der erste Parameter gleich dem ersten vorbestimmten Wert oder in jedem ersten Abschnitt der zweiten Schneidkante der zweite Parameter gleich dem dritten vorbestimmten Wert.

Anders ausgedrückt sind innerhalb der ersten Abschnitte der ersten Schneidkante der erste Parameter konstant, nämlich insbesondere minimal, und innerhalb der ersten Abschnitte der zweiten Schneidkante der zweite Parameter konstant, nämlich insbesondere minimal.

Bei einer medizinischen Schere, wie sie hier beschrieben ist, sind der erste vorbestimmte Wert und der dritte vorbestimmte Wert insbesondere gleich.

Bei einer medizinischen Schere, wie sie hier beschrieben ist, ist insbesondere zumindest entweder in jedem zweiten Abschnitt der ersten Schneidkante der erste Parameter gleich dem zweiten vorbestimmten Wert oder in jedem zweiten Abschnitt der zweiten Schneidkante der zweite Parameter gleich dem vierten vorbestimmten Wert.

Anders ausgedrückt sind innerhalb der zweiten Abschnitte der ersten Schneidkante der erste Parameter konstant, nämlich insbesondere maximal, und innerhalb der zweiten Abschnitte der zweiten Schneidkante der zweite Parameter konstant, nämlich insbesondere maximal.

Bei einer medizinischen Schere, wie sie hier beschrieben ist, sind der zweite vorbestimmte Wert und der vierte vorbestimmte Wert insbesondere gleich.

Bei einer medizinischen Schere, wie sie hier beschrieben ist, ist insbesondere in jedem ersten Abschnitt der ersten Schneidkante der Keilwinkel der ersten Schneidkante nicht größer als 20 Grad oder als 30 Grad oder als 40 Grad, wobei in jedem ersten Abschnitt der zweiten Schneidkante der Keilwinkel der zweiten Schneidkante nicht größer ist als 20 Grad oder als 30 Grad oder als 40 Grad.

Bei einer medizinischen Schere, wie sie hier beschrieben ist, ist insbesondere in jedem zweiten Abschnitt der ersten Schneidkante der Keilwinkel der ersten Schneidkante nicht kleiner als 60 Grad oder als 70 Grad oder als 80 Grad, wobei in jedem zweiten Abschnitt der zweiten Schneidkante der Keilwinkel der zweiten Schneidkante nicht kleiner ist als 60 Grad oder als 70 Grad oder als 80 Grad.

Bei einer medizinischen Schere, wie sie hier beschrieben ist, ist insbesondere in jedem ersten Abschnitt der ersten Schneidkante die Breite der Biseau-Fläche der ersten Schneidkante nicht größer als ein Zwanzigstel oder als ein Fünfzigstel oder als ein Hundertstel der Länge der ersten Schneidkante, wobei in jedem ersten Abschnitt der zweiten Schneidkante die Breite der Biseau-Fläche der zweiten Schneidkante nicht größer ist als ein Zwanzigstel oder als ein Fünfzigstel oder als ein Hundertstel der Länge der zweiten Schneidkante.

Bei einer medizinischen Schere, wie sie hier beschrieben ist, ist insbesondere in jedem zweiten Abschnitt der ersten Schneidkante die Breite der Biseau-Fläche an der ersten Schneidkante nicht kleiner als ein Zwanzigstel oder als ein Zehntel der Länge der ersten Schneidkante, wobei in jedem zweiten Abschnitt der zweiten Schneidkante die Breite der Biseau-Fläche an der zweiten Schneidkante nicht kleiner ist als ein Zwanzigstel oder als ein Zehntel der Länge der zweiten Schneidkante.

Bei einer medizinischen Schere, wie sie hier beschrieben ist, weist eine von der Freifläche abgewandte Seite des ersten Scherenblatts insbesondere Nuten auf, die im Wesentlichen orthogonal zu der ersten Schneidkante orientiert sind, wobei die ersten Abschnitte der ersten Schneidkante jeweils bei einer Nut vorliegen, wobei die zweiten Abschnitte der ersten Schneidkante jeweils zwischen benachbarten Nuten vorliegen.

Eine Nut ist im Wesentlichen orthogonal zu einer Schneidkante, wenn die Nut mit der Schneidkante einen Winkel von mindestens 60 Grad oder von mindestens 70 Grad oder von mindestens 80 Grad einschließt. Jede Nut kann eine konstante oder zumindest über einen Abschnitt konstante oder im Wesentlichen konstante Tiefe aufweisen. Alternativ kann jede Nut eine variierende Tiefe, insbesondere eine von der Schneidkante weg zunächst innerhalb einer vergleichsweise kurzen Strecke zunehmende und danach innerhalb einer vergleichsweise langen Strecke abnehmende Tiefe aufweisen. Jede Nut kann gerade oder gekrümmt verlaufen und einen konstanten oder einen variierenden Querschnitt aufweisen.

Insbesondere weisen die Querschnitte aller Nuten an der von der Freifläche abgewandten Seite des ersten Scherenblatts gleiche oder ähnliche Querschnitte auf. Die Querschnitte der Nuten an der von der Freifläche abgewandten Seite des ersten Scherenblatts können entlang des Scherenblatts von Nut zu Nut gleichmäßig variieren.

Nuten an einer von der Freifläche abgewandten Seite eines Scherenblatts können einerseits besonders einfach herstellbar sein und andererseits die Eigenschaft der Schneidkante des Scherenblatts wirksam räumlich modulieren.

Bei einer medizinischen Schere, wie sie hier beschrieben ist, weist der Querschnitt einer Nut insbesondere einen geraden Randabschnitt auf, der parallel zu der ersten Schneidkante ist.

Insbesondere geht an der ersten Schneidkante der gerade Randabschnitt des Querschnitts der Nut in die erste Schneidkante über.

Bei einer medizinischen Schere, wie sie hier beschrieben ist, weist ein zwischen benachbarten Nuten verbleibender Steg insbesondere einen gerundeten Querschnitt auf.

Ein gerundeter Querschnitt weist insbesondere Krümmungsradien auf, die nicht kleiner als ein Drittel oder als ein Viertel der linearen Abmessungen des Querschnitts sind.

Bei einer medizinischen Schere, wie sie hier beschrieben ist, weist eine von der Freifläche abgewandte Seite des zweiten Scherenblatts insbesondere Nuten auf, die im Wesentlichen orthogonal zu der zweiten Schneidkante orientiert sind, wobei die ersten Abschnitte der zweiten Schneidkante jeweils bei einer Nut vorliegen, und wobei die zweiten Abschnitte der zweiten Schneidkante jeweils zwischen benachbarten Nuten vorliegen.

Bei einer medizinischen Schere, wie sie hier beschrieben ist, weist der Querschnitt einer Nut insbesondere einen geraden Randabschnitt auf, der parallel zu der zweiten Schneidkante ist.

Bei einer medizinischen Schere, wie sie hier beschrieben ist, weist ein zwischen benachbarten Nuten verbleibender Steg insbesondere einen eckigen Querschnitt auf.

Eine oder alternativ mehrere Ecken des Querschnitts des Stegs zwischen benachbarten Nuten und eine bzw. mehrere entsprechende konvexe Kanten des Stegs können ein Gleiten von Gewebe entlang der Schneidkante formschlüssig unterbinden oder behindern.

Bei einer medizinischen Schere, wie sie hier beschrieben ist, weist eine von der Freifläche abgewandte Seite des ersten Scherenblatts insbesondere mehrere erste Oberflächenbereiche, die Teilbereiche einer ersten ebenen oder schwach gekrümmten Fläche sind, und mehrere zweite Oberflächenbereiche, die Teilbereiche einer zweiten ebenen oder schwach gekrümmten Fläche sind, auf, wobei jeder erste Abschnitt der ersten Schneidkante bei einem ersten Oberflächenbereich des ersten Scherenblatt vorliegt und jeder zweite Abschnitt der ersten Schneidkante bei einem zweiten Oberflächenbereich des ersten Scherenblatts vorliegt.

Die von der Freifläche abgewandte Seite des ersten Scherenblatts grenzt in der ersten Schneidkante an die Freifläche des ersten Scherenblatts an. Wenn die ersten Oberflächenbereiche Teilbereiche einer ersten schwach gekrümmten Fläche sind, sind die Krümmungsradien der ersten schwach gekrümmten Fläche insbesondere durchgehend größer oder viel größer als die Länge des ersten Scherenblatts. Wenn die zweiten Oberflächenbereiche in einer zweiten schwach gekrümmten Fläche liegen, sind die Krümmungsradien der zweiten schwach gekrümmten Fläche insbesondere größer oder viel größer als die Länge des ersten Scherenblatts.

Die ersten Oberflächenbereiche des ersten Scherenblatts sind insbesondere erste Teilbereiche der Biseau-Fläche des ersten Scherenblatts, in denen der Keilwinkel oder der Schneidwinkel verhältnismäßig klein ist. Die zweiten Oberflächenbereiche des ersten Scherenblatts sind insbesondere zweite Teilbereiche der Biseau-Fläche des ersten Scherenblatts, in denen der Keilwinkel oder der Schneidwinkel verhältnismäßig groß ist.

Bei einer medizinischen Schere, wie sie hier beschrieben ist, schneiden die Freifläche, die erste ebene oder schwach gekrümmte Fläche, in der die ersten Oberflächenbereiche des ersten Scherenblatts liegen, und die zweite Ebene oder schwach gekrümmte Fläche, in der die zweiten Oberflächenbereiche des ersten Scherenblatts liegen, einander insbesondere in der ersten Schneidkante.

Die ersten Abschnitte der ersten Schneidkante sind dann Bereiche der Schneidkante, in der die ersten Oberflächenbereiche an die Freifläche grenzen, und die zweiten Abschnitte der ersten Schneidkante sind dann Bereiche, in denen die zweiten Oberflächenbereiche an die Freifläche grenzen.

Bei einer medizinischen Schere, wie sie hier beschrieben ist, weist eine von der Freifläche abgewandte Seite des zweiten Scherenblatts insbesondere mehrere erste Oberflächenbereiche, die Teilbereiche einer dritten ebenen oder schwach gekrümmten Fläche sind, und mehrere zweite Oberflächenbereiche, die Teilbereiche einer vierten ebenen oder schwach gekrümmten Fläche sind, auf, wobei die ersten Abschnitte der zweiten Schneidkante jeweils bei einem ersten Oberflächenbereich des zweiten Scherenblatts vorliegen und die zweiten Abschnitte der zweiten Schneidkante jeweils bei einem zweiten Oberflächenbereich des zweiten Scherenblatts vorliegen.

Bei einer medizinischen Schere, wie sie hier beschrieben ist, schneiden die Freifläche, die dritte ebene oder schwach gekrümmte Fläche, in der die ersten Oberflächenbereiche des zweiten Scherenblatts liegen, und die vierte ebene oder schwach gekrümmte Fläche, in der die zweiten Oberflächenbereiche des zweiten Scherenblatts liegen, einander insbesondere in der zweiten Schneidkante.

Die ersten Oberflächenbereiche des zweiten Scherenblatts sind insbesondere erste Teilbereiche der Biseau-Fläche des zweiten Scherenblatts, in denen der Keilwinkel oder der Schneidwinkel verhältnismäßig klein ist. Die zweiten Oberflächenbereiche des zweiten Scherenblatts sind insbesondere zweite Teilbereiche der Biseau-Fläche des zweiten Scherenblatts, in denen der Keilwinkel oder der Schneidwinkel verhältnismäßig groß ist.

Ein medizinisches Instrument für mikroinvasive Anwendungen umfasst einen Schaft, dessen proximales Ende mit einer Handhabungseinrichtung mechanisch verbunden oder verbindbar ist, und eine medizinische Schere, wie sie hier beschrieben ist, die mit einem distalen Ende des Schafts mechanisch verbunden oder verbindbar ist.

Der Schaft ist insbesondere lang und dünn. Die Länge des Schafts ist insbesondere mindestens zehnmal so groß wie der Durchmesser des Schafts. Der Schaft kann gerade oder gekrümmt, starr oder teilweise oder vollständig flexibel ausgebildet sein.

### Kurzbeschreibung der Figuren

Nachfolgend werden Ausführungsformen anhand der beigefügten Figuren näher erläutert. Es zeigen:
- Figur 1: eine schematische Darstellung eines mikroinvasiven medizinischen Instruments;
- Figur 2: eine schematische axonometrische Darstellung einer medizinischen Schere;
- Figur 3: eine weitere schematische axonometrische Darstellung der medizinischen Schere aus Figur 2;
- Figur 4: eine weitere schematische axonometrische Darstellung eines Scherenblatts der medizinischen Schere aus den Figuren 2 und 3;
- Figur 5: eine weitere schematische axonometrische Darstellung der medizinischen Schere aus den Figuren 2 bis 4;
- Figur 6: eine weitere schematische axonometrische Darstellung der medizinischen Schere aus den Figuren 2 bis 5;
- Figur 7: schematische Darstellungen zweier Schnitte durch die Scherenblätter der medizinischen Schere aus den Figuren 2 bis 6;
- Figur 8: schematische Darstellungen zweier Schnitte durch eine andere Ausführungsform einer medizinischen Schere;
- Figur 9: eine schematische axonometrische Darstellung eines Scherenblatts der Ausführungsform der Figur 8.

### Beschreibung von Ausführungsformen

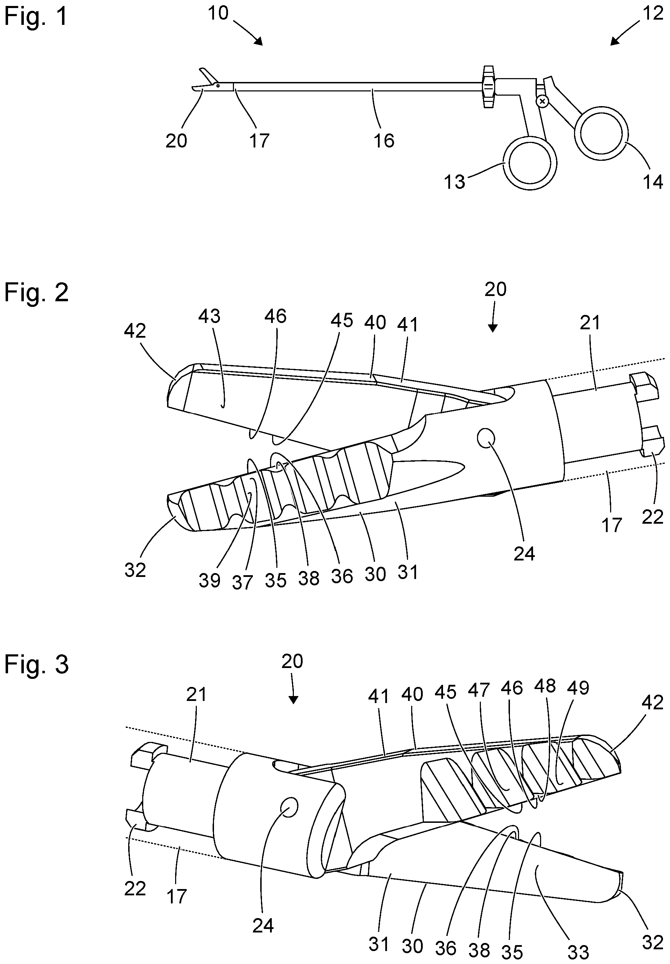

Figur 1 zeigt eine schematische Darstellung eines mikroinvasiven medizinischen Instruments 10, dessen proximales Ende durch eine Handhabungseinrichtung 12 mit einem ersten Griffteil 13 und einem zweiten Griffteil 14 gebildet wird. Das erste Griffteil 13 der Handhabungseinrichtung 12 ist mit einem proximalen Ende eines Schafts 16 starr, insbesondere jedoch zerstörungsfrei lösbar mechanisch verbunden. Ein distales Ende 17 des Schafts 16 ist mit einer medizinischen Schere 20 als Werkzeug mechanisch starr, insbesondere jedoch zerstörungsfrei lösbar verbunden.

Figur 2 zeigt eine schematische und vergrößerte axonometrische Darstellung einer medizinischen Schere 20. Die medizinische Schere kann Teil des medizinischen Instruments 10 aus Figur 1 oder Teil eines anderen medizinischen Instruments sein.

Ein proximales Ende 21 der medizinischen Schere 20 umfasst Knaggen 22 zur zerstörungsfrei lösbaren mechanischen Verbindung (in Gestalt einer Renkverbindung oder Bajonettverbindung) mit einem in Figur 2 nur in gestrichelten Konturen angedeuteten distalen Ende 17 eines Schafts eines medizinischen Instruments, beispielsweise des anhand der Figur 1 dargestellten medizinischen Instruments.

Die medizinische Schere 20 weist ferner ein erstes Scherenblatt 30 auf, dessen proximales Ende 31 bei dem dargestellten Beispiel starr mit dem proximalen Ende der medizinischen Schere 20 und damit starr, wenn auch zerstörungsfrei lösbar, mit dem distalen Ende 17 des Schafts des medizinischen Instruments mechanisch verbunden ist. Bei dem dargestellten Beispiel ist das erste Scherenblatt 30 sogar monolithisch mit dem proximalen Ende 21 der medizinischen Schere 20 und den Knaggen 22 ausgebildet.

Zwischen dem proximalen Ende 31 und dem distalen Ende 32 des ersten Scherenblatts 30 erstreckt sich eine erste Schneidkante 35, 36 zwischen einer bei der Darstellung in Figur 2 vom Betrachter abgewandten und deshalb nicht sichtbaren Freifläche und einer dem Betrachter zugewandten und mehrteiligen Biseau-Fläche 37, 38.

Die erste Schneidkante 35, 36 weist abwechselnd erste Abschnitte 35 und zweite Abschnitte 36 auf. Die ebenen Böden breiter und flacher Nuten 39 bilden erste Teilbereiche 37 der Biseau-Flächen. Diese ersten Teilbereiche 37 bilden mit der Freifläche die ersten Abschnitte 35 der ersten Schneidkante und schließen mit der Freifläche einen vergleichsweise kleinen Keilwinkel ein. Die Nuten 39 sind näherungsweise orthogonal zu der Schneidkante 35, 36 angeordnet.

Schräge Endflächen von Stegen zwischen den Nuten 39 bilden zweite Teilbereiche 38 der Biseau-Fläche. Diese zweiten Teilbereiche 38 bilden mit der Freifläche die zweiten Abschnitte 36 der ersten Schneidkante und schließen mit der Freifläche einen vergleichsweise großen Keilwinkel ein. Der Keilwinkel zwischen Freifläche und Biseau-Fläche 37, 38 des ersten Scherenblatts 30 ist also an den ersten Abschnitten 35 deutlich kleiner als an den zweiten Abschnitten 36 der ersten Schneidkante.

Bei dem dargestellten Beispiel haben die Stege zwischen den Nuten 39 deutlich abgerundete Querschnitte. Bei dem dargestellten Beispiel liegen die ersten Teilbereiche 37 der Biseau-Fläche gemeinsam in einer ersten Ebene oder einer ersten schwach gekrümmten Fläche und die zweiten Teilbereiche 38 der Biseau-Fläche gemeinsam in einer zweiten Ebene oder einer zweiten schwach gekrümmten Fläche. Die erste Fläche und die zweite Fläche sind insofern schwach gekrümmt, als Ihre Krümmungsradien deutlich größer sind als die Länge des ersten Scherenblatts.

Die medizinische Schere 20 umfasst ferner ein zweites Scherenblatt 40 mit einem proximalen Ende 41, das mit dem proximalen Ende 21 der medizinischen Schere und mit dem ersten Scherenblatt 30 über ein Gelenk 24 schwenkbar mechanisch verbunden ist. Das Gelenk 24 definiert eine Schwenkachse orthogonal oder im Wesentlichen orthogonal zu der ersten Schneidkante 35, 36 des ersten Scherenblatts 30. In dem Schaft 16 des medizinischen Instruments 10 ist eine in Figur 2 nicht dargestellte Übertragungseinrichtung, insbesondere eine Übertragungsstange angeordnet, die das zweite Griffteil 14 der Handhabungseinrichtung 12 (vgl. Figur 1) mit dem zweiten, schwenkbaren Scherenblatt 40 der medizinischen Schere 20 derart koppelt, dass eine Bewegung des zweiten Griffteils 14 relativ zu dem ersten Griffteil 13 mit einer Schwenkbewegung des zweiten Scherenblatts 40 relativ zu dem ersten Scherenblatt 30 einhergeht.

Das zweite Scherenblatt 40 weist eine ähnliche Gestalt auf wie das erste Scherenblatt 30. Insbesondere weist das zweite Scherenblatt 40 eine zweite Schneidkante 45, 46 auf, die sich von dem proximalen Ende 41 zu dem distalen Ende 42 des zweiten Scherenblatts 40 erstreckt. Die zweite Schneidkante 45, 46 liegt zwischen einer bei der Darstellung in Figur 2 dem Betrachter zugewandten Freifläche 43 und einer mehrteiligen, bei der Darstellung in Figur 2 vom Betrachter abgewandten und deshalb nicht sichtbaren Biseau-Fläche.

Die erste Schneidkante 35, 36 an dem ersten Scherenblatt 30 und die zweite Schneidkante 45, 46 an dem zweiten Scherenblatt 40 berühren sich in genau einem Schnittpunkt 50. Dies wird durch das Gelenk 24 als Führungseinrichtung und eine leichte vorspannende Krümmung von einem oder beiden Scherenblättern 30, 40 in deren Längsrichtung sichergestellt. Diese Längskrümmung ist in Figur 2 nicht erkennbar. Diese Längskrümmung und die daraus resultierende Vorspannung sind insbesondere so gewählt, dass die Kraft zwischen den Schneidkanten 35, 36, 45, 46 näherungsweise konstant, von dem Ort des Schnittpunkts 50 (vgl. Figuren 2, 3) unabhängig ist.

Figur 3 zeigt eine weitere schematische axonometrische Darstellung der medizinischen Schere 20 aus Figur 2. Die Darstellung in Figur 3 unterscheidet sich von der Darstellung in Figur 2 durch eine andere, näherungsweise entgegengesetzte Blickrichtung. Dadurch sind in Figur 3 die Freifläche 33 an dem ersten Scherenblatt 30 und Biseau-Flächen 47, 48 des zweiten Scherenblatts 40 dem Betrachter zugewandt und sichtbar.

Ähnlich wie die erste Schneidkante 35, 36 an dem ersten Scherenblatt 30 weist auch die zweite Schneidkante 45, 46 an dem zweiten Scherenblatt 40 abwechselnd erste Abschnitte 45 mit kleinem Keilwinkel zwischen der Freifläche 43 (vgl. Figur 2) und dem angrenzenden ersten Teilbereich 47 der Biseau-Fläche und zweite Abschnitte 46 mit großem Keilwinkel zwischen der Freifläche 43 und dem angrenzenden zweiten Teilbereich 48 der Biseau-Fläche auf.

Auch an dem zweiten Scherenblatt 40 bilden ebene Bodenflächen breiter und flacher Nuten 49 die ersten Teilbereiche 47 der Biseau-Fläche mit einem vergleichsweise kleinen Keilwinkel zur Freifläche. Stirnflächen an den Enden von Stegen zwischen den Nuten 49 bilden zweite Teilbereiche 48 der Biseau-Fläche. Diese zweiten Teilflächen 48 der Biseau-Fläche schließen mit der Freifläche 43 (vgl. Figur 2) einen vergleichsweise großen Keilwinkel ein. Diese zweiten Teilbereiche 48 der Biseau-Fläche bilden mit der Freifläche die zweiten Abschnitte 46 der zweiten Schneidkante und schließen mit der Freifläche einen vergleichsweise großen Keilwinkel ein. Der Keilwinkel zwischen Freifläche und Biseau-Fläche 45, 46 des zweiten Scherenblatts 40 ist also an den ersten Abschnitten 45 deutlich kleiner als an den zweiten Abschnitten 46 der zweiten Schneidkante.

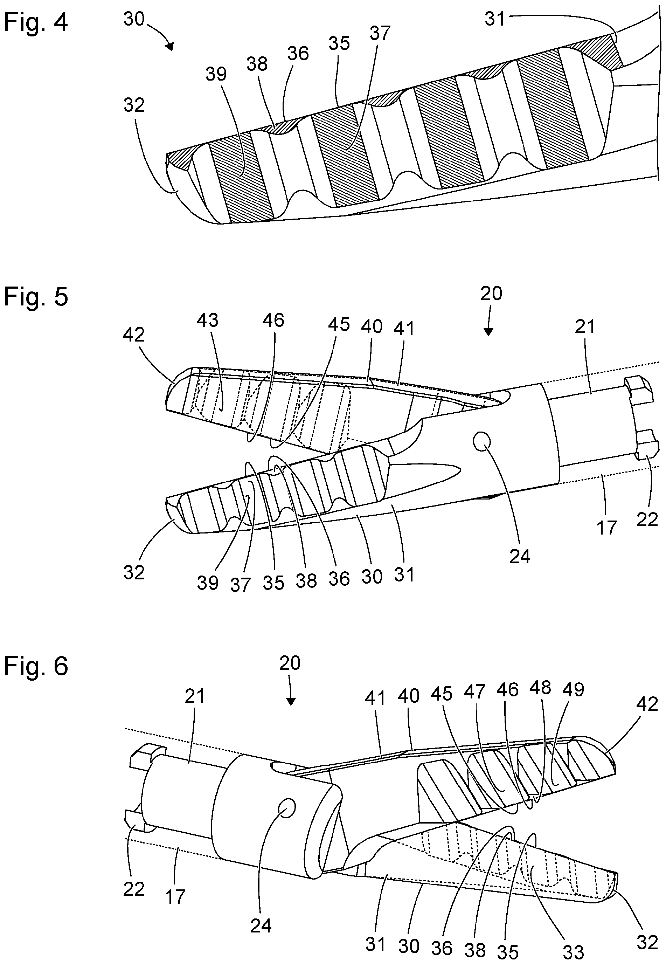

Figur 4 zeigt eine weitere, vergrößerte schematische axonometrische Darstellung des ersten Scherenblatts 30 der medizinischen Schere aus den Figuren 2 und 3. Die Darstellung in Figur 4 unterscheidet sich von der Darstellung in Figur 2 dadurch, dass die an die ersten Abschnitte 35 der ersten Schneidkante angrenzenden ersten Teilbereiche 37 der Biseau-Fläche und die an die zweiten Abschnitte 36 der ersten Schneidkante angrenzenden Teilbereiche 38 der Biseau-Fläche unterschiedlich schraffiert und damit deutlich unterscheidbar sind.

Bei dem dargestellten Beispiel liegen alle ersten Teilflächen 37 der Biseau-Fläche in einer ersten schwach gekrümmten Fläche und alle zweiten Teilflächen 38 der Biseau-Fläche in einer zweiten schwach gekrümmten Fläche. Die schwache Krümmung der ersten Fläche und der zweiten Fläche ist in Figur 4 nicht erkennbar. Sie resultiert insbesondere aus der bereits erwähnten, in Figur 4 ebenfalls nicht erkennbaren leichten Krümmung des ersten Scherenblatts 30 in seiner Längsrichtung. Die Freifläche 33 (vgl. Figur 3), die erste Fläche, in der die ersten Teilbereiche 37 der Biseau-Fläche liegen, und die zweite Fläche, in der die zweiten Teilflächen 38 der Biseau-Fläche liegen, können jeweils in einer oder in mehreren Richtungen gekrümmt oder alternativ eben sein.

Die erste Schneidkante 35, 36 des ersten Scherenblatts 30 ist die - schwach gekrümmte oder gerade - Schnittlinie der Freifläche mit der ersten Fläche, in der die ersten Teilbereiche 37 der Biseau-Fläche liegen, und gleichzeitig mit der zweiten Fläche, in der die zweiten Teilbereiche 38 der Biseau-Fläche liegen.

Figur 5 zeigt eine weitere schematische axonometrische Darstellung der medizinischen Schere 20 aus den Figuren 2 bis 4. Die Darstellung in Figur 5 ähnelt weitgehend der Darstellung in Figur 2. Die Darstellung in Figur 5 unterscheidet sich von der Darstellung in Figur 2 dadurch, dass die Strukturen an der vom Betrachter abgewandten Seite des zweiten Scherenblatts 40 in gestrichelten Linien angedeutet sind.

Figur 6 zeigt eine weitere schematische axonometrische Darstellung der medizinischen Schere 20 aus den Figuren 2 bis 5. Die Darstellung in Figur 6 ähnelt weitgehend der Darstellung in Figur 3. Die Darstellung in Figur 6 unterscheidet sich von der Darstellung in Figur 3 dadurch, dass die Strukturen an der vom Betrachter abgewandten Seite des ersten Scherenblatts 30 durch gestrichelte Linien angedeutet sind.

In den Figuren 5 und 6 ist erkennbar, dass die Nuten 39 und damit auch die ersten Teilbereiche 37 der Biseau-Fläche an dem ersten Scherenblatt 30 gegenüber den Nuten 49 und damit auch den ersten Teilbereichen 47 der Biseau-Fläche an dem zweiten Scherenblatt 40 versetzt angeordnet sind. Jedem ersten Abschnitt 35 der ersten Schneidkante mit kleinem Keilwinkel an dem ersten Scherenblatt 30 liegt ein zweiter Abschnitt 46 der zweiten Schneidkante mit großem Keilwinkel an dem zweiten Scherenblatt 40 gegenüber. Jedem ersten Abschnitt 45 der zweiten Schneidkante mit kleinem Keilwinkel an dem zweiten Scherenblatt 40 liegt ein zweiter Abschnitt 36 der ersten Schneidkante mit großem Keilwinkel an dem ersten Scherenblatt 30 gegenüber.

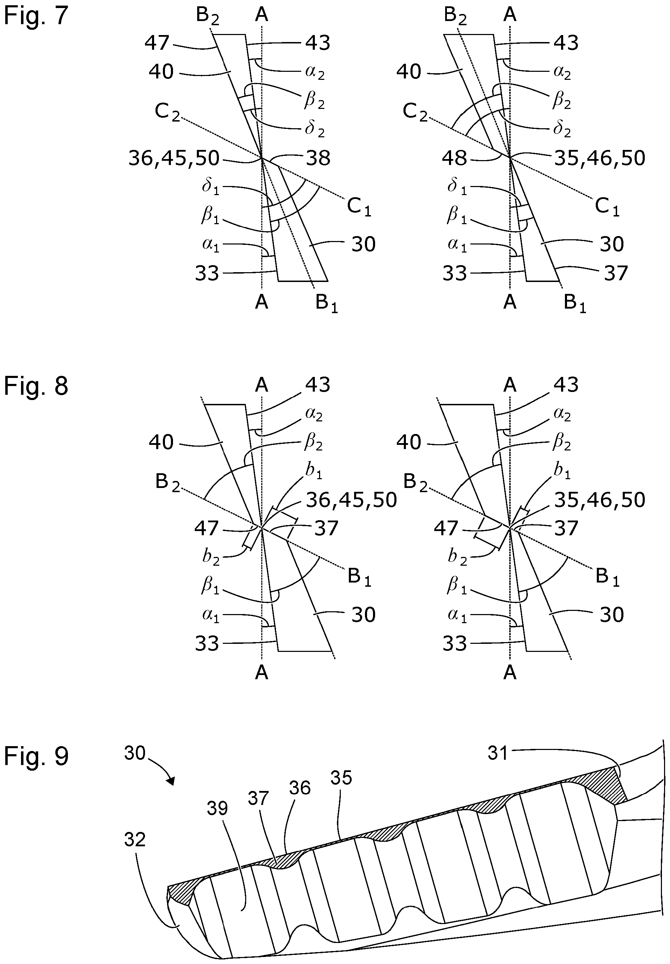

Figur 7 zeigt eine schematische Darstellung zweier Schnitte durch die medizinische Schere 20 aus den Figuren 2 bis 6. Die Schnittebenen der Figur 7 sind näherungsweise parallel zu der durch das Gelenk 24 (vgl. Figuren 2 bis 6) definierten Schwenkachse und näherungsweise orthogonal zu den Schneidkanten 35, 36, 45, 46. Die Schnittebene der Figur 7 enthält den Schnittpunkt 50 der Schneidkanten 35, 36, 45, 46.

Die Schneidebene A ist die Ebene orthogonal zu der durch das Gelenk 24 definierten Schwenkachse (vgl. Figuren 2 bis 6), in der die Schneidkanten 35, 36, 45, 46 relativ zueinander bewegt werden.

Die Freifläche 33 des ersten Scherenblatts 30 schließt mit der Schneidebene oder Schnittebene A den Freiwinkel *α*₁, ein. Die Freifläche 43 des zweiten Scherenblatts 43 schließt mit der Schneidebene A den Freiwinkel *α*₂ ein. Gestrichelte Linien deuten die Positionen und Orientierungen der erwähnten ersten Flächen B₁, B₂, in denen die ersten Teilbereiche 37, 47 der Biseau-Flächen der Scherenblätter 30, 40 liegen, und die Positionen und Orientierungen der erwähnten zweiten Flächen C₁, C₂, in denen die zweiten Teilbereiche 38, 48 der Biseau-Flächen der Scherenblätter 30, 40 liegen, an.

In Figur 7 links dargestellt ist eine Situation oder Konfiguration der medizinischen Schere, bei der der Schnittpunkt 50 der ersten Schneidkante an dem ersten Scherenblatt 30 und der zweiten Schneidkante an dem zweiten Scherenblatt 40 zwischen einem zweiten Abschnitt 36 der ersten Schneidkante mit einem großen Wert des Keilwinkels *β*₁ (und damit auch des Schneidwinkels *δ*₁) und einem ersten Abschnitt 45 der zweiten Schneidkante mit einem kleinen Wert des Keilwinkels *β*₂ (und damit auch des Schneidwinkels *δ*₂) liegt - ähnlich wie bei der in den Figuren 2, 3, 5, 6 gezeigten Situation.

In Figur 7 rechts dargestellt ist eine Situation oder Konfiguration der medizinischen Schere, bei der der Schnittpunkt 50 der ersten Schneidkante an dem ersten Scherenblatt 30 und der zweiten Schneidkante an dem zweiten Scherenblatt 40 zwischen einem ersten Abschnitt 35 der ersten Schneidkante mit einem kleinen Wert des Keilwinkels *β*₁ (und damit auch des Schneidwinkels *δ*₁) und einem zweiten Abschnitt 46 der zweiten Schneidkante mit einem großen Wert des Keilwinkels *β*₂ (und damit auch des Schneidwinkels δ₂) liegt.

Beim Schließen der medizinischen Schere wechseln sich die in Figur 7 links und rechts dargestellten Situationen ab, so dass meist und nur immer kurzzeitig unterbrochen an dem momentanen Schnittpunkt 50 der Schneidkanten 35, 36, 45, 46 einer der beiden Keilwinkel *β*₁, *β*₂ einen kleinen Wert aufweist.

Figur 8 zeigt eine schematische Darstellung zweier Schnitte durch eine alternative Ausführungsform einer medizinischen Schere, die in einigen Merkmalen, Eigenschaften und Funktionen der anhand der Figuren 2 bis 7 dargestellten medizinischen Schere ähnelt. Die Art der Darstellung, insbesondere die Schnittebenen, entspricht derjenigen der Figur 7.

Nachfolgend sind insbesondere Merkmale, Eigenschaften und Funktionen der medizinischen Schere beschrieben, in denen diese sich von der anhand der Figuren 2 bis 7 dargestellten medizinischen Schere unterscheidet.

Die in Figur 8 gezeigte Ausführungsform unterscheidet sich von der anhand der Figuren 2 bis 7 dargestellten medizinischen Schere insbesondere dadurch, dass die Keilwinkel *β*₁, *β*₂ entlang den Schneidkanten 35, 36, 45, 46 konstant sind oder nur langsam variieren. Stattdessen variieren der Breiten *b*₁, *b*₂ der Biseau-Flächen 37, 47.

In Figur 8 links dargestellt ist eine Situation, bei der der Schnittpunkt 50 der Schneidkanten an einem zweiten Abschnitt 36 der ersten Schneidkante mit einem vergleichsweise großen Wert der Breite *b*₁ der Biseau-Fläche 37 des ersten Scherenblatts 30 und an einem ersten Abschnitt 45 der zweiten Schneidkante mit einem vergleichsweise kleinen Wert der Breite *b*₂ der Biseau-Fläche 47 an dem zweiten Scherenblatt 40 anliegt, *b*₁ > *b₂.*

In Figur 8 rechts dargestellt ist eine Situation oder Konfiguration der medizinischen Schere, bei der der Schnittpunkt 50 der Schneidkanten an einem ersten Abschnitt 35 der ersten Schneidkante mit einem vergleichsweise kleinen Wert der Breite *b*₁ der Biseau-Fläche 37 des ersten Scherenblatts 30 und an einem zweiten Abschnitt 46 der zweiten Schneidkante mit einer vergleichsweise großen Wert der Breite *b*₂ der Biseau-Fläche 47 an dem zweiten Scherenblatt 40 anliegt, *b*₁ < *b₂.*

Figur 9 zeigt eine schematische axonometrische Darstellung des ersten Scherenblatts 30 der anhand der Figur 8 dargestellten medizinischen Schere. Die Art der Darstellung entspricht derjenigen der Figur 4.

In Figur 9 ist erkennbar, dass die schraffiert dargestellte Biseau-Fläche 37 des ersten Scherenblatts 30 durchgehend ist, jedoch abwechselnd sehr schmale und deutlich breitere Bereiche aufweist. Das zweite Scherenblatt 40 (vgl. Figur 8) ist insbesondere ähnlich gestaltet.

Die Scherenblätter 30, 40 können insbesondere ähnlich wie bei der anhand der Figuren 2 bis 7 dargestellten medizinischen Schere durch Erzeugen mehrerer breiter und flacher Nuten 39 strukturiert werden. Anders als bei der anhand der Figuren 2 bis 7 dargestellten medizinischen Schere berühren die Nuten 39 die Freifläche 33 (vgl. Figur 8) jedoch nicht, sondern sind von dieser beabstandet, so dass an keiner Stelle eine Nut die Biseau-Fläche 37 bildet. Vielmehr modulieren die Nuten 39 lediglich die Breite der Biseau-Fläche 37.

Ähnlich wie bei der anhand der Figuren 2 bis 7 dargestellten medizinischen Schere sind auch bei der anhand der Figuren 8, 9 dargestellten medizinischen Schere die Nuten in den Scherenblättern 30, 40 derart versetzt angeordnet, dass beim Schließen der medizinischen Schere der Schnittpunkt 50 der Schneidkanten jederzeit an mindestens einem Abschnitt 35, 45 einer Schneidkante, bei dem die Breite *b*₁, *b*₂ der Biseau-Fläche 37, 47 kleiner als ein vorbestimmter Wert ist, anliegt.

### Bezugszeichen

- 10: Medizinisches Instrument für mikroinvasive Anwendungen
- 12: Handhabungseinrichtung des medizinischen Instruments 10
- 13: erstes Griffteil der Handhabungseinrichtung 12
- 14: zweites Griffteil der Handhabungseinrichtung 12
- 16: Schaft des medizinischen Instruments 10
- 17: distales Ende des Schafts 16
- 20: medizinische Schere des medizinischen Instruments 10
- 21: proximales Ende der medizinischen Schere 20, zur insbesondere lösbaren starren mechanischen Verbindung mit dem distalen Ende 17 des Schafts 16
- 22: Knagge an dem proximalen Ende 21 der medizinischen Schere 20, zur Bildung einer Renkverbindung zwischen der medizinischen Schere 20 und dem distalen Ende 17 des Schafts 16
- 24: Gelenk der medizinischen Schere 20
- 30: erstes, insbesondere feststehendes Scherenblatt der medizinischen Schere 20
- 31: proximales Ende des ersten Scherenblatts 30
- 32: distales Ende des ersten Scherenblatts 30
- 33: Freifläche des ersten Scherenblatts 30
- 35: erster Abschnitt einer ersten Schneidkante
- 36: zweiter Abschnitt der ersten Schneidkante
- 37: Biseau-Fläche des ersten Scherenblatts 30 oder **erster Teilbereich der Biseau-Fläche** an dem ersten Abschnitt 35 der ersten Schneidkante
- 38: zweiter Teilbereich der Biseau-Fläche des ersten Scherenblatts 30 an dem zweiten Abschnitt 36 der ersten Schneidkante
- 39: Nut an der von der Freifläche 33 abgewandten Seite des ersten Scherenblatts 30
- 40: zweites, insbesondere schwenkbares Scherenblatt der medizinischen Schere 20
- 41: proximales Ende des zweiten Scherenblatts 40
- 42: distales Ende des zweiten Scherenblatts 40
- 43: Freifläche des zweiten Scherenblatts 40
- 45: erster Abschnitt einer zweiten Schneidkante
- 46: zweiter Abschnitt der zweiten Schneidkante
- 47: Biseau-Fläche des zweiten Scherenblatts 40 oder erster Teilbereich der Biseau-Fläche an dem ersten Abschnitt 45 der zweiten Schneidkante
- 48: zweiter Teilbereich der Biseau-Fläche des zweiten Scherenblatts 40 an dem zweiten Abschnitt 46 der zweiten Schneidkante
- 49: Nut an der von der Freifläche 43 abgewandten Seite des zweiten Scherenblatts 40
- 50: Schnittpunkt der ersten Schneidkante 35, 36 und der zweiten Schneidkante 45, 46
- A: Schneidebene oder Schnittfläche
- B₁: erste ebene oder schwach gekrümmte Fläche, in der die ersten Teilbereiche 37 der Biseau-Fläche des ersten Scherenblatts 30 liegen
- B₂: erste ebene oder schwach gekrümmte Fläche, in der die ersten Teilbereiche 47 der Biseau-Fläche des zweiten Scherenblatts 40 liegen
- C₁: zweite ebene oder schwach gekrümmte Fläche, in der die zweiten Teilbereiche 38 der Biseau-Fläche des ersten Scherenblatts 30 liegen
- C₂: zweite ebene oder schwach gekrümmte Fläche, in der die zweiten Teilbereiche 48 der Biseau-Fläche des zweiten Scherenblatts 40 liegen
- *α*₁: Freiwinkel der Freifläche 33 an dem ersten Scherenblatt 30
- *β*₁: Keilwinkel zwischen der Freifläche 33 und der Biseau-Fläche an dem ersten Scherenblatt 30
- *δ*₁: Schneidwinkel an dem ersten Scherenblatt 30, *δ*₁ = *α*₁ + *β*₁
- *b*₁: Breite der Biseau-fläche 37, 38 des ersten Scherenblatts
- *α*₂: Freiwinkel der Freifläche 43 an dem zweiten Scherenblatt 40
- *β*₂: Keilwinkel zwischen der Freifläche 43 und der Biseau-Fläche an dem zweiten Scherenblatt 40
- *δ*₂: Schneidwinkel an dem zweiten Scherenblatt 40, *δ*₂ = *α*₂ + *β*₂
- *b*₂: Breite der Biseau-Fläche 47, 48 des zweiten Scherenblatts

## Patentansprüche

1. **Medizinische Schere** (20) für mikroinvasive Anwendungen, mit:
einem **ersten Scherenblatt** (30) mit einer ersten Schneidkante (35, 36) zwischen einer Freifläche (33) und einer Biseau-Fläche (37, 38);
einem relativ zu dem ersten Scherenblatt (30) bewegbaren **zweiten Scherenblatt** (40) mit einer zweiten Schneidkante (45, 46) zwischen einer Freifläche (43) und einer Biseau-Fläche (47, 48);
einer **Führungseinrichtung** (24) zur mechanischen Führung des zweiten Scherenblatts (40) relativ zu dem ersten Scherenblatt (30) derart, dass die zweite Schneidkante (45, 46) die erste Schneidkante (35, 36) jederzeit in einem Schnittpunkt (50) berührt,
wobei die Führungseinrichtung (24) **mit einem Schaft** (16) eines mikroinvasiven Instruments (10) starr verbunden oder **verbindbar** ist,
wobei die erste Schneidkante (35, 36) durch einen ersten Parameter (*β*₁, *δ*₁, *b*₁) charakterisiert ist, der entlang der ersten Schneidkante (35, 36) mehrfach zunimmt und mehrfach abnimmt,
wobei die zweite Schneidkante (45, 46) durch einen zweiten Parameter (*β*₂, *δ*₂, *b₂)* charakterisiert ist, der entlang der zweiten Schneidkante (45, 46) mehrfach zunimmt und mehrfach abnimmt,
wobei der erste Parameter und der zweite Parameter jeweils der **Keilwinkel** (*β*₁, *β*₂) zwischen Freifläche (33, 43) und Biseau-Fläche (37, 38, 47, 48) oder der **Schneidwinkel** (*δ*₁, *δ*₂) oder die **Breite** (*b*₁, *b*₂) **der Biseau-Fläche** (37, 38, 47, 48) ist,
**dadurch gekennzeichnet, dass**
die Führungseinrichtung und die Schneidkanten so ausgebildet und angeordnet sind, dass die Schneidkanten einander **jederzeit in genau einem Schnittpunkt** berühren.

2. Medizinische Schere (20) nach dem vorangehenden Anspruch, bei der
die erste Schneidkante abwechselnd **erste Abschnitte** (35), in denen der erste Parameter (*β*₁, *δ*₁, *b*₁) **nicht größer** ist als ein erster vorbestimmter Wert, und **zweite Abschnitte** (36), in denen der erste Parameter (*β*₁, *δ*₁, *b*₁) **nicht kleiner** ist als ein zweiter vorbestimmter Wert, der größer ist als der erste vorbestimmte Wert, aufweist, die zweite Schneidkante abwechselnd **erste Abschnitte** (45), in denen der zweite Parameter (*β*₂, *δ*₂, *b*₂) **nicht größer** ist als ein dritter vorbestimmter Wert, und **zweite Abschnitte** (46), in denen der zweite Parameter (*β*₂, *δ₂*, *b*₂) **nicht kleiner** ist als ein vierter vorbestimmter Wert, der größer ist als der dritte vorbestimmte Wert, aufweist,
wobei jeder zweite Abschnitt (36) der ersten Schneidkante einem ersten Abschnitt (45) der zweiten Schneidkante gegenüberliegt und jeder zweite Abschnitt (46) der zweiten Schneidkante einem ersten Abschnitt (35) der ersten Schneidkante gegenüberliegt.

3. Medizinische Schere (20) nach dem vorangehenden Anspruch, bei der
die ersten Abschnitte (35) und die zweiten Abschnitte (36) der ersten Schneidkante und die ersten Abschnitte (45) und die zweiten Abschnitte (46) der zweiten Schneidkante so angeordnet sind, dass beim Schließen der medizinischen Schere (20) der Schnittpunkt (50) **jederzeit zumindest entweder** an einem **ersten Abschnitt** (35) der ersten Schneidkante oder an einem **ersten Abschnitt** (45) der zweiten Schneidkante liegt.

4. Medizinische Schere (20) nach Anspruch 2, bei der
die ersten Abschnitte (35) und die zweiten Abschnitte (36) der ersten Schneidkante und die ersten Abschnitte (45) und die zweiten Abschnitte (46) der zweiten Schneidkante so angeordnet sind, dass beim Schließen der medizinischen Schere (20) Bereiche, in denen der Schnittpunkt (50) **weder** an einem **ersten Abschnitt** (35) der ersten Schneidkante noch an einem **ersten Abschnitt** (45) der zweiten Schneidkante liegt, nicht größer sind als jeweils unmittelbar benachbarte Bereiche, in denen der Schnittpunkt (50) an einem **ersten Abschnitt** (35) der ersten Schneidkante oder an einem **ersten Abschnitt** (45) der zweiten Schneidkante liegt.

5. Medizinische Schere (20) nach einem der Ansprüche 2 bis 4, bei der zumindest entweder
in jedem ersten Abschnitt (35) der ersten Schneidkante der erste Parameter (*β*₁, *δ*₁, *b*₁) **gleich** dem ersten vorbestimmter Wert ist oder
in jedem zweiten Abschnitt (45) der zweiten Schneidkante der zweite Parameter (*β*₂, *δ*₂, *b*₂) **gleich** dem dritten vorbestimmter Wert ist.

6. Medizinische Schere (20) nach dem vorangehenden Anspruch, bei der
der **erste vorbestimmte Wert** und der **dritte vorbestimmte Wert gleich** sind.

7. Medizinische Schere (20) nach einem der Ansprüche 2 bis 6, bei der eine von der Freifläche (33) abgewandte Seite des ersten Scherenblatts (30) **Nuten** (39) aufweist, die im Wesentlichen orthogonal zu der ersten Schneidkante (35, 36) orientiert sind,
die ersten Abschnitte (35) der ersten Schneidkante jeweils bei einer Nut (39) vorliegen,
die zweiten Abschnitte (36) der ersten Schneidkante jeweils zwischen benachbarten Nuten (39) vorliegen.

8. Medizinische Schere (20) nach dem vorangehenden Anspruch, bei der
der Querschnitt einer Nut (39) einen geraden Randabschnitt aufweist, der parallel zu der ersten Schneidkante (35, 36) ist.

9. Medizinische Schere (20) nach einem der Ansprüche 7 und 8, bei der ein zwischen benachbarten Nuten (39) verbleibender Steg einen gerundeten Querschnitt aufweist.

10. Medizinische Schere (20) nach einem der Ansprüche 2 bis 9, bei der
eine von der Freifläche (43) abgewandte Seite des zweiten Scherenblatts (40) **Nuten** (49) aufweist, die im Wesentlichen orthogonal zu der zweiten Schneidkante (45, 46) orientiert sind,
die ersten Abschnitte (45) der zweiten Schneidkante jeweils bei einer Nut (49) vorliegen,
die zweiten Abschnitte (46) der zweiten Schneidkante jeweils zwischen benachbarten Nuten (49) vorliegen.

11. Medizinische Schere (20) nach einem der vorangehenden Ansprüche, bei der
eine von der Freifläche (33) abgewandte Seite des ersten Scherenblatts (30) mehrere erste Oberflächenbereiche (37), die Teilbereiche einer ersten ebenen oder schwach gekrümmten Fläche sind, und mehrere zweite Oberflächenbereiche (38), die Teilbereiche einer zweiten ebenen oder schwach gekrümmten Fläche sind, aufweist,
jeder erste Abschnitte (35) der ersten Schneidkante bei einem ersten Oberflächenbereich (37) des ersten Scherenblatts (30) vorliegt,
jeder zweiten Abschnitte (36) der ersten Schneidkante bei einem zweiten Oberflächenbereich (38) des ersten Scherenblatts (30) vorliegt.

12. Medizinische Schere (20) nach dem vorangehenden Anspruch, bei der die Freifläche (33), die erste ebene oder schwach gekrümmte Fläche, in der die ersten Oberflächenbereiche (37) liegen, und die zweite ebene oder schwach gekrümmte Fläche, in der die zweiten Oberflächenbereiche (38) liegen, einander in der ersten Schneidkante (35, 36) schneiden.

13. **Medizinisches Instrument** (10) für mikroinvasive Anwendungen, mit:
einem **Schaft** (16), dessen proximales Ende mit einer Handhabungseinrichtung (12) mechanisch verbunden oder verbindbar ist,
einer **medizinische Schere** (20) nach einem der vorangehenden Ansprüche, die mit einem distalen Ende (17) des Schafts (16) mechanisch verbunden oder verbindbar ist.

## Claims

1. Medical scissors (20) for micro-invasive applications, comprising:
a first scissor blade (30) with a first cutting edge (35, 36) between a flank surface (33) and a bevel surface (37, 38);
a second scissor blade (40), which is movable relative to the first scissor blade (30) and has a second cutting edge (45, 46) between a flank surface (43) and a bevel surface (47, 48);
a guide device (24) for mechanically guiding the second scissor blade (40) relative to the first scissor blade (30) in such a way that the second cutting edge (45, 46) touches the first cutting edge (35, 36) at any time at an intersection point (50), wherein the guide device (24) is rigidly connected or connectable to a shaft (16) of a micro-invasive instrument (10),
wherein the first cutting edge (35, 36) is distinguished by a first parameter (*β₁*, *δ₁, b₁*) which increases several times and decreases several times along the first cutting edge (35, 36),
wherein the second cutting edge (45, 46) is distinguished by a second parameter (*β₂*, *δ₂, b₂*) which increases several times and decreases several times along the second cutting edge (45, 46),
wherein the first parameter and the second parameter are in each case the wedge angle (*β₁, β₂*) between flank surface (33, 43) and bevel surface (37, 38, 47, 48) or the cutting angle (*δ₁*, *δ₂*) or the width (*b₁, b₂*) of the bevel surface (37, 38, 47, 48),
**characterized in that** the guide device and the cutting edges are designed and arranged such that the cutting edges touch each other at any time at exactly one intersection point.

2. Medical scissors (20) according to the preceding claim, in which the first cutting edge has alternately first portions (35), in which the first parameter (β1, δ1, b1) is not greater than a first predetermined value, and second portions (36), in which the first parameter δ1, b1) is not smaller than a second predetermined value, which is greater than the first predetermined value, the second cutting edge has alternately first portions (45), in which the second parameter (β2, δ2, b2) is not greater than a third predetermined value, and second portions (46) in which the second parameter (β2, δ2, b2) is not smaller than a fourth predetermined value, which is greater than the third predetermined value,
wherein each second portion (36) of the first cutting edge lies opposite a first portion (45) of the second cutting edge, and each second portion (46) of the second cutting edge lies opposite a first portion (35) of the first cutting edge.

3. Medical scissors (20) according to the preceding claim, in which the first portions (35) and the second portions (36) of the first cutting edge and the first portions (45) and the second portions (46) of the second cutting edge are arranged such that, upon closing of the medical scissors (20), the intersection point (50) at any time lies at least either on a first portion (35) of the first cutting edge or on a first portion (45) of the second cutting edge.

4. Medical scissors (20) according to Claim 2, in which the first portions (35) and the second portions (36) of the first cutting edge and the first portions (45) and the second portions (46) of the second cutting edge are arranged such that, upon closing of the medical scissors (20), regions in which the intersection point (50) lies neither on a first portion (35) of the first cutting edge nor on a first portion (45) of the second cutting edge are not larger than respective directly adjacent regions in which the intersection point (50) lies on a first portion (35) of the first cutting edge or on a first portion (45) of the second cutting edge.

5. Medical scissors (20) according to one of Claims 2 to 4, in which at least either
the first parameter (*β₁*, *δ₁*, *b₁*) is identical to the first predetermined value in each first portion (35) of the first cutting edge, or
the second parameter (*β₂, δ₂, b₂*) is identical to the third predetermined value in each second portion (45) of the second cutting edge.

6. Medical scissors (20) according to the preceding claim, in which the first predetermined value and the third predetermined value are identical.

7. Medical scissors (20) according to one of Claims 2 to 6, in which
a side of the first scissor blade (30) facing away from the flank surface (33) has grooves (39) oriented substantially orthogonally to the first cutting edge (35, 36),
the first portions (35) of the first cutting edge are each present at a groove (39),
the second portions (36) of the first cutting edge are each present between adjacent grooves (39).

8. Medical scissors (20) according to the preceding claim, in which the cross section of a groove (39) has a straight edge portion that is parallel to the first cutting edge (35, 36).

9. Medical scissors (20) according to either of Claims 7 and 8, in which a web that remains between adjacent grooves (39) has a rounded cross section.

10. Medical scissors (20) according to one of Claims 2 to 9, in which
a side of the second scissor blade (40) facing away from the flank surface (43) has grooves (49) oriented substantially orthogonally to the second cutting edge (45, 46),
the first portions (45) of the second cutting edge are each present at a groove (49),
the second portions (46) of the second cutting edge are each present between adjacent grooves (49).

11. Medical scissors (20) according to one of the preceding claims, in which
a side of the first scissor blade (30) facing away from the flank surface (33) has a plurality of first surface regions (37), which are sub-regions of a first flat or slightly curved surface, and a plurality of second surface regions (38), which are sub-regions of a second flat or slightly curved surface,
each first portion (35) of the first cutting edge is present at a first surface region (37) of the first scissor blade (30),
each second portion (36) of the first cutting edge is present at a second surface region (38) of the first scissor blade (30).

12. Medical scissors (20) according to the preceding claim, in which the flank surface (33), the first flat or slightly curved surface, in which the first surface regions (37) lie, and the second flat or slightly curved surface, in which the second surface regions (38) lie, intersect one another in the first cutting edge (35, 36).

13. Medical instrument (10) for micro-invasive applications, comprising:
a shaft (16), of which the proximal end is connected or connectable to a handling device (12),
medical scissors (20) according to one of the preceding claims, which are connected or connectable mechanically to a distal end (17) of the shaft (16).

## Revendications

1. **Ciseaux médicaux** (20) pour applications micro-invasives, avec :
une **première lame de ciseaux** (30) avec un premier bord de coupe (35, 36) entre une surface libre (33) et une surface de biseau (37, 38) ;
une **deuxième lame de ciseaux** (40) déplaçable par rapport à la première lame de ciseaux (30), avec un deuxième bord de coupe (45, 46) entre une surface libre (43) et une surface de biseau (47, 48) ;
un **dispositif de guidage** (24) pour guider mécaniquement la deuxième lame de ciseaux (40) par rapport à la première lame de ciseaux (30) de telle sorte que le deuxième bord de coupe (45, 46) est en contact avec le premier bord de coupe (35, 36) à tout moment en un point d'intersection (50),
le dispositif de guidage (24) étant relié ou **pouvant être relié** de manière rigide **à une tige** (16) d'un instrument micro-invasif (10),
le premier bord de coupe (35, 36) étant **caractérisé par** un premier paramètre (*β₁, δ₁, b₁*) qui augmente plusieurs fois et diminue plusieurs fois le long du premier bord de coupe (35, 36),
le deuxième bord de coupe (45, 46) étant **caractérisé par** un deuxième paramètre (*β₂, δ₂, b₂*) qui augmente plusieurs fois et diminue plusieurs fois le long du deuxième bord de coupe (45, 46),
le premier paramètre et le deuxième paramètre étant respectivement **l'angle de coin** (*β₁, β₂*) entre la surface libre (33, 43) et la surface de biseau (37, 38, 47, 48) ou **l'angle de coupe** (*δ₁, δ₂*) ou **la largeur** (*b₁, b₂*) de la **surface de biseau** (37, 38, 47, 48),
**caractérisé en ce que**
le dispositif de guidage et les bords de coupe sont configurés et agencés de telle sorte que les bords de coupe sont en contact l'un avec l'autre **à tout moment en exactement un point d'intersection.**

2. Ciseaux médicaux (20) selon la revendication précédente, dans lesquels le premier bord de coupe présente en alternance des **premières sections** (35), dans lesquelles le premier paramètre (*β₁, δ₁, b₁*) n'est **pas supérieur** à une première valeur prédéterminée, et des **deuxièmes sections** (36), dans lesquelles le premier paramètre (*β₁, δ₁, b₁*) n'est **pas inférieur** à une deuxième valeur prédéterminée qui est supérieure à la première valeur prédéterminée, le deuxième bord de coupe présente en alternance des **premières sections** (45) dans lesquelles le deuxième paramètre (*β₂, δ₂, b₂*) n'est **pas supérieur** à une troisième valeur prédéterminée, et des **deuxièmes sections** (46) dans lesquelles le deuxième paramètre (*β₂*, *δ₂, b₂*) n'est **pas inférieur** à une quatrième valeur prédéterminée qui est supérieure à la troisième valeur prédéterminée,
chaque deuxième section (36) du premier bord de coupe étant opposée à une première section (45) du deuxième bord de coupe et chaque deuxième section (46) du deuxième bord de coupe étant opposée à une première section (35) du premier bord de coupe.

3. Ciseaux médicaux (20) selon la revendication précédente, dans lesquels les premières sections (35) et les deuxièmes sections (36) du premier bord de coupe et les premières sections (45) et les deuxièmes sections (46) du deuxième bord de coupe sont agencées de telle sorte que, lors de la fermeture des ciseaux médicaux (20), le point d'intersection (50) se situe **à tout moment au moins soit** sur une **première section** (35) du premier bord de coupe soit sur une **première section** (45) du deuxième bord de coupe.

4. Ciseaux médicaux (20) selon la revendication 2, dans lesquels
les premières sections (35) et les deuxièmes sections (36) du premier bord de coupe et les premières sections (45) et les deuxièmes sections (46) du deuxième bord de coupe sont agencées de telle sorte que, lors de la fermeture des ciseaux médicaux (20), des zones, dans lesquelles le point d'intersection (50) ne se situe **ni** sur une **première section** (35) du premier bord de coupe ni sur une **première section** (45) du deuxième bord de coupe, ne sont pas plus grandes que des zones immédiatement voisines dans lesquelles le point d'intersection (50) se situe sur une **première section** (35) du premier bord de coupe ou sur une **première section** (45) du deuxième bord de coupe.

5. Ciseaux médicaux (20) selon l'une quelconque des revendications 2 à 4, dans lesquels au moins soit
dans chaque première section (35) du premier bord de coupe, le premier paramètre (*β₁, δ₁, b₁*) est **égal** à la première valeur prédéterminée, soit
dans chaque deuxième section (45) du deuxième bord de coupe, le deuxième paramètre (*β₂, δ₂, b₂*) est **égal** à la troisième valeur prédéterminée.

6. Ciseaux médicaux (20) selon la revendication précédente, dans lesquels
la **première valeur prédéterminée** et la **troisième valeur prédéterminée** sont **égales.**

7. Ciseaux médicaux (20) selon l'une quelconque des revendications 2 à 6, dans lesquels
un côté de la première lame de ciseaux (30) détourné de la surface libre (33) présente des **rainures** (39), qui sont orientées essentiellement orthogonalement par rapport au premier bord de coupe (35, 36),
les premières sections (35) du premier bord de coupe se trouvent chacune au niveau d'une rainure (39),
les deuxièmes sections (36) du premier bord de coupe se trouvent chacune entre des rainures voisines (39).

8. Ciseaux médicaux (20) selon la revendication précédente, dans lesquels
la section transversale d'une rainure (39) présente une section de bord droite qui est parallèle au premier bord de coupe (35, 36).

9. Ciseaux médicaux (20) selon l'une quelconque des revendications 7 et 8, dans lesquels une nervure restant entre des rainures voisines (39) présente une section transversale arrondie.

10. Ciseaux médicaux (20) selon l'une quelconque des revendications 2 à 9, dans lesquels
un côté de la deuxième lame de ciseaux (40) détourné de la surface libre (43) présente des **rainures** (49), qui sont orientées essentiellement orthogonalement par rapport au deuxième bord de coupe (45, 46),
les premières sections (45) du deuxième bord de coupe se trouvent chacune au niveau d'une rainure (49),
les deuxièmes sections (46) du deuxième bord de coupe se trouvent chacune entre des rainures voisines (49).

11. Ciseaux médicaux (20) selon l'une quelconque des revendications précédentes, dans lesquels
un côté de la première lame de ciseaux (30) détourné de la surface libre (33) présente plusieurs premières zones de surface (37) qui sont des zones partielles d'une première surface plane ou faiblement incurvée, et plusieurs deuxièmes zones de surface (38) qui sont des zones partielles d'une deuxième surface plane ou faiblement incurvée,
chaque première section (35) du premier bord de coupe se trouve au niveau d'une première zone de surface (37) de la première lame de ciseaux (30),
chaque deuxième section (36) du premier bord de coupe se trouve au niveau d'une deuxième zone de surface (38) de la première lame de ciseaux (30).

12. Ciseaux médicaux (20) selon la revendication précédente, dans lesquels la surface libre (33), la première surface plane ou faiblement incurvée dans laquelle se situent les premières zones de surface (37) et la deuxième surface plane ou faiblement incurvée dans laquelle se situent les deuxièmes zones de surface (38) se coupent mutuellement dans le premier bord de coupe (35, 36).

13. **Instrument médical** (10) pour applications micro-invasives, avec :
une **tige** (16), dont l'extrémité proximale est reliée ou peut être reliée mécaniquement à un dispositif de manipulation (12),
des **ciseaux médicaux** (20) selon l'une quelconque des revendications précédentes, qui sont reliés ou peuvent être reliés mécaniquement à une extrémité distale (17) de la tige (16).
